# EUROPEAN PATENT SPECIFICATION

(11) **EP 1 541 552 B1**
(45) Date of publication and mention of the grant of the patent: **14.11.2007**
(21) Application number: 03028159.6
(22) Date of filing: 08.12.2003
(51) Int. Cl.: C07D 207/46, C07D 211/94, C07D 263/04, C07B 63/00, C07B 41/00

(54) **Process for the recovery of nitroxy compounds from organic solutions and oxidation process**
Verfahren zur Rückgewinnung von Nitroxy-Verbindungen aus organischen Lösungen und Oxidationsverfahren
Procédé de récupération de composés nitroxy à partir de solutions organiques et procédé d'oxydation

(43) Date of publication of application: 15.06.2005
(73) Proprietor: SCA Hygiene Products AB, 405 03 Göteborg (SE)
(72) Inventor: Jetten, Jan, 3701 JL Zeist (NL); Besemer, Arie, 3958 CC Amerongen (NL)
(74) Representative: HOFFMANN EITLE

(56) References cited:
- WO-A-96/36621
- WO-A-02/058844
- US-A1- 2002 151 431
- NOOY DE A E J ET AL: "ON THE USE OF STABLE ORGANIC NITROXYL RADICALS FOR THE OXIDATION OFPRIMARY AND SECONDARY ALCOHOLS" SYNTHESIS, GEORG THIEME VERLAG. STUTTGART, DE, 1 October 1996 (1996-10-01), pages 1153-1174, XP002072173 ISSN: 0039-7881

## Description

The present invention relates to a process for the recovery of nitroxy compounds from organic solutions or aqueous/organic solutions and a corresponding process for the oxidation of hydroxy compounds in the presence of nitroxy compounds wherein the nitroxy compounds recovered thereby can be recycled to the oxidation step.

### Background art

Nitroxy-mediated oxidations are a useful tool to convert primary alcohols to aldehydes and/or carboxylic acids or secondary alcohols to ketones. Especially the oxidation by means of TEMPO (2,2,6,6-tetramethylpiperidine-N-oxyl, formula I in the scheme below) has attracted a lot of interest over the last years since it allows the selective introduction of aldehyde and/or carboxylic acids into alcohol substrates such as polysaccharides.

The reaction can be conducted in three ways. First, a nitrosonium ion (III), which is the actual active species can be generated catalytically in situ from a nitroxide in the presence of a primary oxidant, such as hypochlorous acid/hypochlorite as shown in the scheme below. Depending on the process conditions, the nitrosonium ion will oxidize the alcohol, i.e. the hydroxy compound, to the corresponding aldehyde (as shown in the scheme) or carboxy compound while being reduced itself to the hydroxylamine. Equimolar amounts of the resulting hydroxylamine and nitrosonium ion will then recombine (synproportionate) under suitable reaction conditions to form the nitroxy compound. Then, the catalytic cycle newly starts.

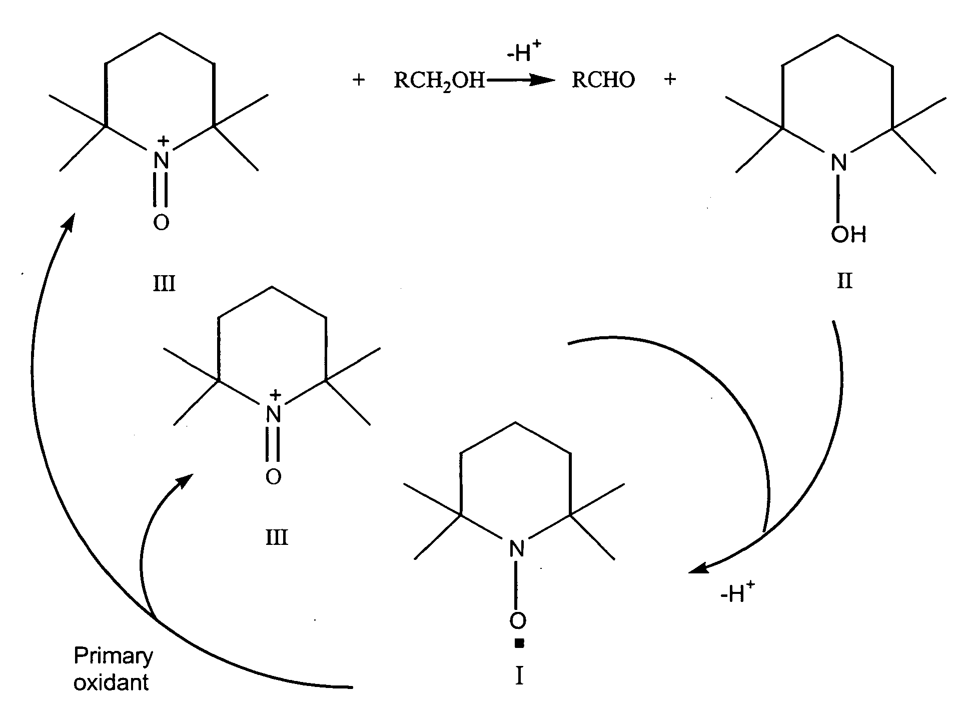

Second, the nitrosonium ion can be generated in situ by a disproportionation reaction of the nitroxy compound (I) to the corresponding hydroxylamine (II) and the nitrosonium ion (III) as shown in the following scheme. As the disproportionation reaction must be conducted under (strongly) acidic conditions, the hydroxylamine (II) is typically present in its protonated form (IV).

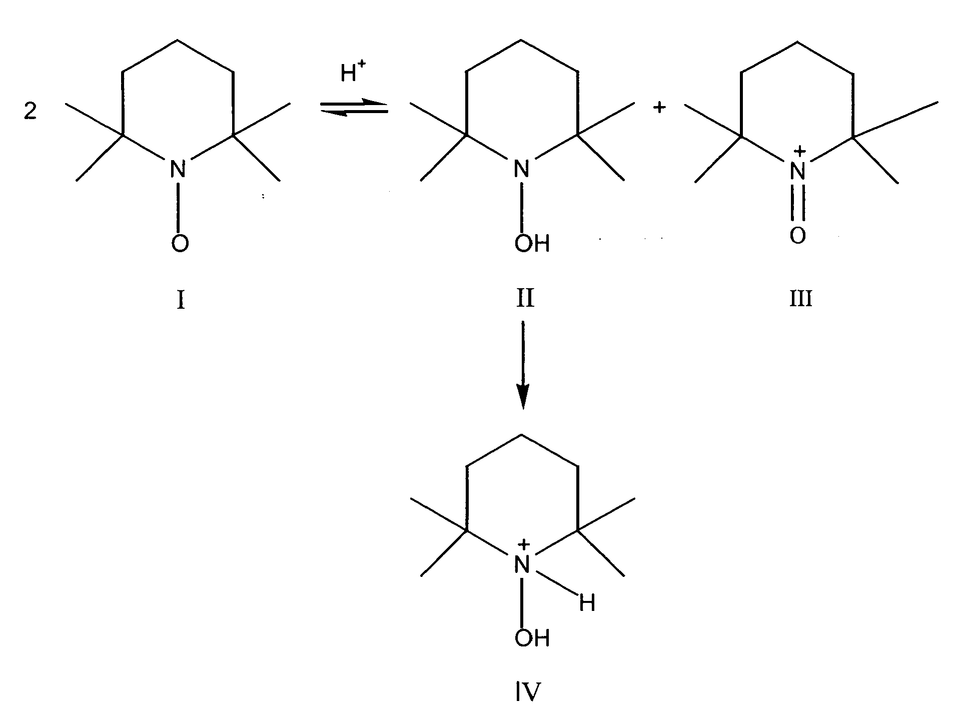

Third, it is also possible to prepare a salt of the nitrosonium ion, which then can be used in stoichiometric amounts.

Moreover, a relatively new technique involves the enzymatic conversion of nitroxy compounds as described in WO 00/50463.

There is extensive patent and scientific literature dealing with the synthesis and the use of nitroxy compounds, mostly TEMPO and its derivatives in oxidation reactions.

Reviews were published for instance by J.M. Bobbitt et al. in heterocycles, vol. 27, No. 2, 1988 "Organic nitrosonium salts as oxidants in organic chemistry" and by A.E.J. De Nooy et al. in J.Synthetic Org. Chem 1996 (10),1153-1174 "on the use of stable organic nitroxyl radicals for the oxidation of primary and secondary alcohols".

If TEMPO and certain related nitroxy compounds are used as a catalyst in the oxidation of alcohols, for instance oligo- or polysaccharides, in aqueous suspensions and solutions, the nitroxy compound is often partially lost during the isolation of the reaction product due to the relatively high volatility of some nitroxy compounds. This is an undesired situation for an economically feasible process. Very few documents deal with technical solutions for overcoming this problem or the recovery of TEMPO and its derivatives in general.

WO 96/36621 proposes in this regard a method for obtaining a catalytically active mixture based on stable nitroxide radicals wherein at least a part of the reaction mixture is subjected to an azeotropic distillation. During the azeotropic distillation step, volatile nitroxide radicals are at least partially distilled over with the liquid medium. However, this method requires distillation devices and creates high costs by energy consumption. Further, it is not applicable to less volatile TEMPO derivatives.

Another technique for avoiding the loss of TEMPO as a catalyst involves affixing the same to a polymer solid phase which then can be easily recovered from the reaction medium (T. Miyazawa et al. in J. Polymer Sci. Polymer Chem. Ed. 23 (1985) 1527-1535 and 2487-2494). Similarly, DE 4209869 A describes a method for immobilizing 4-hydroxy-TEMPO on polyvinyl-benzyl.

US 2003/0073871 pertains to the continuous oxidation of alcohol substrates to aldehydes in the presence of nitroxy compounds in an alkaline 2-phase system. The nitroxy compound can be present in the organic phase, in the aqueous phase or bound to solid supports.

However, the reaction rate of immobilized organic nitroxy compounds is typically lower than in homogeneous systems.

WO 02/59064 A1 relates to various recovery processes for TEMPO and related nitroxy compounds based on hydrophobic interactions. One option involves bringing into contact the nitroxy compound with hydrophobic resins such as XAD resins, for instance by passing the reaction mixture over a chromatographic column filled with one of these resins. The hydrophobic character of TEMPO and other organic nitroxy compounds leads to a strong affinity to hydrophobic resins allowing the separation from the reaction mixture. Nevertheless, this recovery process creates new problems insofar as, after extracting the organic nitroxy compound from the column, the organic solvent (eluent) has to be removed by evaporation. At this point in time, the high volatility of some organic nitroxy compounds such as TEMPO or 3,4- dehydro-TEMPO leads again to an undesired loss of this costly material.

WO 02/58844 describes a special group of nitroxy compounds wherein 4 TEMPO molecules are integrated into a heterocyclic molecule and their use in the oxidation of alcohol substrates. These nitroxy compounds can be recovered by filtration or water evaporation steps. One method for the recovery of the nitroxy compound catalyst involves the filtration at pH values above 8. According to another embodiment the organic phase containing the catalyst is stirred with concentrated hydrochloric acid (HCl) to transfer the catalyst into the aqueous phase followed by evaporating the water under formation of the catalyst salt.

Further, it is known in the art that N-methylmorpholine-N-oxide (NMMO) which is used as solvent in the Lyocell process can be recovered and purified by means of anion exchange resins (H.Mertel et al., Papier (Darmstadt), 46 (3), 101-5, 1992, "Purification, recovery and methods for quantitative determination of N-methylmorpholine-N-oxide". The separation of tertiary amine oxides, such as NMMO from aqueous solutions by means of cationic exchange resins having carboxylate anchor groups is further known from EP 0 468 951 A1.

In view of the above, there is a strong need for an efficient recovery process for TEMPO and related organic nitroxy compounds.

Therefore, it is one object of the present invention to provide a process for the efficient recovery of TEMPO and related organic nitroxy compounds.

It is a further object of the present invention to provide a process allowing the recovery of TEMPO and related organic nitroxy compounds from organic or organic/aqueous solutions of the type obtained in chromatographic separation processes.

It is a further object of the present invention to increase the efficiency of a chromatographic separation process for TEMPO and related organic nitroxy compound using organic or organic/aqueous solvents as eluent.

It is one further technical object of the present invention to provide a process for the recovery of TEMPO and related organic nitroxy compounds, which can be integrated in a process for the oxidation of organic hydroxy compounds in the presence of nitroxy compounds in order to recycle the recovered nitroxy compounds to this oxidation step.

It is also an object of the present invention to provide a recovery process for TEMPO and related nitroxy compounds wherein the loss of nitroxy compound due to its volatility is minimized.

### Brief description of the invention

The above technical objects are solved by the following processes:
1. Process for the recovery of an organic nitroxy compound from a solution of this nitroxy compound in an organic solvent or a mixture of a water-miscible organic solvent and water, said process comprising the steps of:
   (a) adjusting the pH of said solution to a value below 4 to generate a nitrosonium ion and the protonated hydroxylamine
   (b) removing the organic solvent until a residue containing the nitrosonium ion and the protonated hydroxylamine has formed,
   (c) if necessary, redissolving the residue obtained in water, and
   (d) neutralizing the resulting aqueous solution to recover the nitroxy compound.
2. Process for the oxidation of a hydroxy compound in the presence of a nitroxy compound comprising the steps of
   (i) oxidizing a hydroxy compound in the presence of a nitroxy compound and optionally a primary oxidant in a water-containing reaction medium,
   (ii) separating the nitroxy compound remaining in the reaction mixture obtained thereby by contacting the reaction mixture with a hydrophobic resin,
   (iii)removing the nitroxy compound from the hydrophobic resin by eluting the same with an organic solvent or a mixture of water and a water-miscible organic solvent, said mixture having a lower polarity than the reaction medium,
   (iv) recovering the nitroxy compound from the resulting solution by the process according to the above process,
   (v) recycling the nitroxy compound obtained thereby into step (i).

### Detailed description of the invention

The **process for the recovery of nitroxy compounds** according to the present invention comprises the four steps (a) to (d).

According to the first **step (a)** of the claimed recovery process, the pH of an organic or organic/aqueous solution containing a nitroxy compound is adjusted to a value below 4, preferably below 3, more preferably below 2 with a suitable acid in order to generate a nitrosonium ion (oxoammonium ion) and the protonated hydroxylamine (throughout the specification and the claims pH values refer to a measurement at 20°C). At these pH values nitroxy compounds undergo a disproportionation reaction leading to the corresponding oxdized species (nitrosonium ion) and the reduced species (hydroxylamine) the latter being automatically protonated. In contrast to the nitroxy compound itself, the generated charged species, i.e. the nitrosonium ion and the protonated hydroxylamine are not volatile. Therefore, in the following step b, the organic solvent can be removed without any substantial loss of material.

In step a, any solvent capable of dissolving organic nitroxy compounds, as described in further detail below, can be used.
Preferably, those solvents are employed that are also suitable as eluent in the chromatographic separation of organic nitroxy compounds by means of hydrophobic resins.
Furthermore, the use of relatively low-boiling solvents having a boiling point of less than 150°C (at ambient pressure of 1013mbar), preferably less than 120°C, in particular less than 100°C is preferred.

Examples of the organic solvents are water-miscible organic solvents, such as water-miscible alcohols (e.g. ethanol, 1-or 2-propanol; t-butanol), ethers, ketones or nitriles preferably non-alcoholic water-miscible solvents such as dioxane, tetrahydrofuran, acetone or acetonitrile (CH₃CN).

The term "water-miscible" is to be understood as complete (concentration-independent) mutual solubility at 20°C.

As far as aqueous/organic solutions are concerned, the content of organic solvent is preferably more than 20 Vol.-%, more preferably more than 50 Vol.-%, even more preferably more than 50 Vol.-%.

For adjusting the pH to acidic values causing disproportionation, any acid can be used, e.g. perchloric acid, although it is generally preferred to employ non-oxidising and non-reducing organic or inorganic acids. In order to reach the necessary low pH values, it is generally advantageous to use stronger acids, for instance those having pK values (at 25°C in an aqueous solution) of less than 5, preferably less than 3, for instance less than 1 in particular less than -1. Very strong acids fulfilling even the latter requirement are sulfuric acid or hydrochloric acid. If the entire recovery and oxidation process is to be conducted under TCF (total chlorine free) conditions, it is preferred to use chlorine-free acids, such as organic sulfonic acids ( e.g. benzenesulfonic acid, toluenesulfonic acid, methyl p-toluenesulfonic acid), sulfuric acid, phosphoric acid or nitric acid.

The content of organic nitroxy compound in the solution is only limited by the solubility or dispersibility. Depending on the type of organic nitroxy compound to be dissolved, this amount preferably ranges from 0.1 to 30 wt%, preferably 1 to 10 wt%, based on the weight of the solvent (mixture). It should be noted that nitrosonium and protonated hydroxylamine easily dissolve in water whereas a slightly lower solubility is observed for the corresponding nitroxy compounds.

Similarly, there are no specific limitations regarding the type of nitroxy compound to be used. The organic nitroxy compound is preferably a sterically hindered organic nitroxy compound (This, as well as the following description of structural features, also naturally applies to the corresponding nitrosonium and hydroxylamine compounds).
Furthermore, the use of secondary nitroxides is preferred (NMMO, N-methyl morpholin-N-oxide is for instance a tertiary nitroxide). One, particularly two bulky groups in the α position to the NO is/are suitable for sterical hindrance of the NO group, e.g. optionally substituted phenyl or aliphatic substituents that are linked to the nitrogen atom of the NO by a quaternary C atom, e.g. tert-butyl. In other words, it is preferred that one, in particular two quaternary carbon atoms are present in α-position of the N-atom. According to preferred embodiments, one, in particular both α-carbon atom are dimethylsubstituted. It is similarly preferred that the nitroxy compound lacks α-hydrogen atoms. Two substituents can also be combined into an alkylen unit optionally interrupted by a hetero-atom (e.g. O,N) (to form an alicyclic or heterocyclic ring). The molecular weight of the nitroxy compound is preferably less than 500, its carbon number preferably less than 40, in particular less than 30, e.g. less than 20.

Preferred oxidation systems can be represented by the following formula (V) 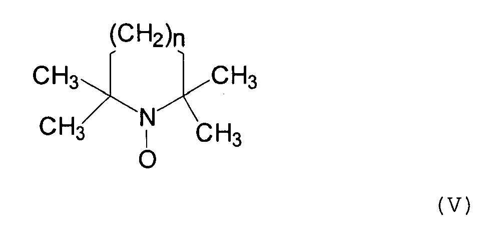 where n = 0 or 1 and where the methylene groups of the ring may carry one or more substituents selected from alkyl, alkoxy, aryl, aryloxy, amino, amido (e.g. acetamido, 2-bromacetamido and 2-iodacetamido), oxo, cyano, hydroxy, carboxyl, phosphonooxy, maleimido, isothiocyanato, alkyloxy, fluorophosphinyloxy (particularly ethoxyfluorophosphinyloxy), substituted or unsubstituted benzoyloxy, e.g. 4-nitrobenzoyloxy. If n = 1 (i.e. the ring represents a piperidine), these groups typically substitute the 4-position of the piperidine. Examples are 4-acetamido-TEMPO, 4-acetoxy-TEMPO or 4-hydroxy-TEMPO. The di-tert.-alkyl nitroxy unit can also be present as part of a polymer structure such as {(CH₃)₂-C-(CH₂)₂₋₃-(CH₃)₂-C-NO-}ₘ-. Hydroxy, amino and amido are preferred among these substituents on account of the stability of the nitroxy compound under acidic conditions. The ring may also be partially unsaturated as in 3,4-dehydro-TEMPO.

An example of n=0 is PROXYL (2,2,5,5-tetramethylpyrrolidine-N-oxyl).

DOXYL (4,4-dimethyloxazolidine-N-oxyl) can also be used as nitroxy compound in the present invention.

According to **step (b)** of the claimed process, the organic solvent is removed until a residue containing the nitrosonium ion and the protonated hydroxylamine has formed. As "residue" we understand the solid or liquid mixture formed after removal of the organic solvent. This residue is typically solid or oily and consists essentially of nitrosonium and protonated hydroxylamine if the starting solution was based on organic solvent(s) only. The residue may also be an aqueous solution or dispersion of nitrosonium ion and protonated hydroxylamine remaining after the removal of an organic solvent from its mixture with water.

The organic solvent can be removed with techniques known in the art. It is preferred to remove the organic solvent by distillation either at normal pressure (1013 mbar) or reduced pressure. As a rule, it is preferred to conduct step (b) at temperatures ranging from 15°C to 90°C. If correspondingly the solvent used has a boiling point at normal pressure above this upper limit of 90°C, the distillation is conducted preferably under reduced pressure.

If step (b) leads to a solid or oily residue, the same is dissolved in water according to **step (c)**. There are no specific restrictions regarding the amount of water to be used for dissolving the residue. Typically and preferably water is added in amounts resulting in concentrations of 0,1 to 30 wt%, preferably 1 to 10 wt% based on the amount of water.

In **step (d)** of the claimed process, the resulting aqueous solution is neutralized. During this step nitrosonium ion and protonated hydroxylamine recombine (synproportionate) to form the nitroxy compound. Then, the same can be newly used in the oxidation of a hydroxy compound. The equilibrium between dis- and synproportionation starts to shift to the synproportionation at pH values of at least 3. It is however preferred to adjust the aqueous solution to pH values of at least 4, more preferably at least 5, while a preferred upper limit is 11.

Any suitable base can be used in step (d) even though, as a rule, oxidizing or reducing bases are to be avoided since these could cause undesired side reactions. Preferred bases are derived from monovalent metals and involve alkali metal bases or basic alkali metal salts such as sodium hydroxide, potassium hydroxide, sodium carbonate, potassium carbonate, sodium hydrogen carbonate, potassium hydrogen carbonate, sodium or potassium acetate.

The nitroxy compound regenerated in step (d) can be newly used in the oxidation of a hydroxy compound.

This leads to **a process for the oxidation of a hydroxy compound** comprising the steps (i) to (v) as laid down in the claims.

According to **step (i)**, the hydroxy compound is oxidized in the presence of an nitroxy compound and optionally a primary oxidant.

According to the present invention, there are no specific limitations regarding the hydroxy compound to be oxidized in step (i). It is one feature of nitroxy-mediated oxidations that they are compatible with a huge variety of different substrate hydroxy compounds. Thus, the hydroxy compound can comprise a primary or secondary hydroxy functionality. The oxidation of primary hydroxy compounds is however preferred since techniques known in the art allow conducting the nitroxy-mediated oxidation of primary hydroxy compounds with particularly high selectivities and conversion rates. The primary hydroxy compound is oxidized to the corresponding aldehyde and/or carboxylic acid. The interruption of the oxidation process and/or a suitable reaction conditions enable the isolation of the intermediate aldehyde.
Interruption of the oxidation process is particularly suited for the oxidation of polysaccharides.

Examples for suitable hydroxy compound substrates are low molecular weight (up to MW 1000) aliphatic or aromatic-aliphatic hydroxy compounds, such as oligosaccharides as well as hydroxy compounds having a higher molecular weight including polymeric hydroxy compounds such as polysaccharides. The oxidation of oligo- or polysaccharides, such as starch or cellulose typically leads to the corresponding C6-aldehyde and/or carboxy derivates. The oxidation of cellulose and cellulose- containing materials, such as pulp is particularly preferred.

The starting pulps which may be used for oxidation may relate to primary fibrous materials (raw pulps) or to secondary fibrous materials, whereby a secondary fibrous material is defined as a fibrous raw material recovered from a recycling process. The primary fibrous materials may relate both to a chemically digested pulp (e.g. Kraft or sulfite pulp) and to mechanical pulp such as thermorefiner mechanical pulp (TMP), chemothermorefiner mechanical pulp (CTMP) or high temperature chemithermomechanical pulp (HTCTMP). Synthetic cellulose-containing fibers can also be used. Preference is nevertheless given to the use of pulp from plant material, particularly wood-forming plants. Fibers of softwood (usually originating from conifers), hardwood (usually originating from deciduous trees) or from cotton linters can be used for example. Fibers from esparto (alfa) grass, bagasse (cereal straw, rice straw, bamboo, hemp), kemp fibers, flax and other woody and cellulosic fiber sources can also be used as raw materials. The corresponding fiber source is chosen in accordance with the desired properties of the end product in a manner known in the art.

The oxidized pulps can be used for the manufacture of paper, in particular tissue paper having improved strength properties. Prior to or after oxidation the starting pulps can be beaten (refined) with the aim of further enhancing paper strength. In the manufacture of oxidized pulps it is further preferred to carry out all oxidation steps in the absence of chlorine-containing oxidants as basis for the production of TCF or ECF paper.

The hydroxy compound to be oxidized is dissolved or dispersed in a suitable reaction medium, for instance water, the already-mentioned water-miscible (preferably non-alcoholic) organic solvents such as THF, dioxane, acetone, CH₃CN or mixtures thereof. The use of water is particularly preferred.

The nitroxy compound can be used stoichiometrically or in combination with a primary oxidizing agent capable of returning the nitroxy compound to the oxidized state after its consumption (reduction) (a list of suitable reaction conditions and compounds is found in A.E.J. De Nooy, Synthesis 1996, 1153-1174). The pH range of the reaction generally varies between 1 and 14, preferably 2 and 12, particularly 4 and 11. For mechanistical reasons, as known in the art, oxidation reactions carried out at lower pH values tend to proceed more slowly. The reaction temperature is preferably between 5°C and 80°C. The nitroxy compound may be added to the hydroxy compound as a solid (also as a pasty substance) or as a solution (usually as an aqueous solution).

In the second case (nitroxy compound + primary oxidizing agent), preferably first the nitroxy compound and then the primary oxidizing agent is added. Suitable oxidants can be selected among primary oxidants typically used together with organic nitroxy compounds such as chlorine, bromine, iodine, hypochlorite, chlorite (in combination with hypochlorite), hypobromite, iodite, Fe(CN)₆ ³⁻ , transition metals of periods Va to VIIIa in the oxidation state of at least +3, oxidases, ozone, hydrogen peroxide, peroxosulfate and/or peracids. For each of these primary oxidants suitable reaction conditions, as known in the art, are to be selected. Thus, it can for instance be undesired to use hypochlorite at pH <2 since then chlorine is formed, and tends to escape from the reaction mixture.

The oxidizing agent can be added all at once or distributed over the duration of the reaction (e.g. by evenly adding it in drops). The primary oxidizing agent (e.g. peracetic acid, ozone, hypohalite, the above metal compounds, enzyme) is preferably used in an amount of 1 to 100 mol%, in relation to the hydroxy functionality to be oxidized. The catalytic quantity of the nitroxy compound is preferably 0,01 to 5 mol% relative to the hydroxy functionality to be oxidized.

In the case of a so-called "stoichiometric" reaction with the nitroxy compound, this compound is preferably used in an amount of 4 to 440 mol.%, relative to the hydroxy functionality to be oxidized. This also includes the possibility of partially oxidizing polyhydroxy compounds such as cellulose present in pulp.

One version of performing the oxidation with the formula (V) nitroxy compound is described in WO 95/07393, which teaches the oxidation with a catalytic amount of the nitroxy compound and a hypohalite (e.g. NaOCl) as a primary oxidizing agent in an aqueous reaction medium at a pH between 9 and 13. Under these conditions, a primary hydroxy group (e.g. of cellulosic pulp) is oxidized via the corresponding aldehyde group into the carboxyl group.

An alternative version lies in the oxidation with a peracid, a precursor or a salt thereof as a primary oxidizing agent in the presence of a catalytic amount of the nitroxy compound (particularly optionally substituted TEMPO) and a catalytic amount of a halide (e.g. NaBr), preferably in the pH range of 2 to 11, particularly 5 to 9. The halide is preferably used in a quantity of 0,1 to 40, particularly 0,5 to 10 mol% relative to the hydroxy groups, e.g. those of cellulosic pulp. In the latter case, the nitroxy compound is preferably used in a quantity of 0,1 to 2,5 wt.%, relative to the dry weight (oven-dried) of the cellulosic pulp.

The peracid is preferably a peralkanoic acid, particularly peracetic acid, or persulfuric acid. Depending on the reaction duration, this embodiment of oxidation leads to aldehydes and/or carboxyl groups, for instance at C(6) of the glucose unit of the cellulose. It is, however, also possible to perform oxidation just using the nitroxy compound (particularly the formula (V) compound) as a mediator and peracid (e.g. persulfuric acid) as an oxidizing agent without halide, particularly bromide. More preferred pH ranges are 6 to 8 for peracetic acid/bromide and 7 to 9 for persulfuric acid, respectively.

Another version lies in the combination of a catalytic amount of the nitroxy compound (particularly optionally substituted TEMPO) and a suitable oxidic compound of a metal of the transition metals of periods Va to VIIIa in the oxidation state of at least +3, e.g. oxides and oxygen-containing ions of manganese, chromium, iron, nickel, ruthenium and vanadium, e.g. vanadium pentoxide, iron oxide, chromium (VI) oxide, chromates and particularly manganese (IV) oxide and salts of permanganic acid. The reaction is preferably conducted at a pH between 5 and 11. The nitroxy compound is preferably used in a molar amount of 0,01 to 5% based on the hydroxy functionality to be oxidized. As to pulp oxidation, an amount of 0,1 to 2,5 wt.%, relative to the dry weight (oven-dried) of the fibrous material is preferred. The reaction temperature is preferably less than 80°C, particularly 30 to 60°C. Depending on the duration of the reaction, this embodiment of the oxidation leads to aldehydes and/or carboxyl groups, for instance at C(6) of cellulosic pulp.

A further version lies in the oxidation of the cellulose with a catalytic amount of the formula (V) nitroxy compound that is hydroxy-, amino- or amido-substituted (e.g. 4-hydroxy TEMPO) at a pH between 1 and 7, particularly 2 to 6. In this version, a hypohalite (e.g. NaOCl) or ozone is particularly suitable as a primary oxidizing agent. The nitroxy compound is preferably used here in an amount of 0,01 to 5 mol% with respect to the hydroxy functionality to be oxidized. In the oxidation of pulp, the use of 0,05 to 15 wt.% nitroxy compound and 0,1 to 20 wt.% primary oxidizing agent, each relative to the dry weight (oven-dried) of the fibrous material is preferred. The reaction temperature is preferably 5 to 50°C. Depending on the reaction duration, this embodiment of oxidation results in aldehydes and/or carboxyl groups, e.g. at C(6) of the glucose unit of the cellulose. Halogen-free acids, such as sulfuric acid or toluene sulfonic acid, are particularly suitable for setting the pH.

According to a preferred embodiment, the nitroxy compound can also be used with enzymes and/or metal complexes. In this embodiment the catalytic amount of the nitroxyl compound is preferably 0,1-2,5 mol% with respect to the C(6)-hydroxy to be oxidized.

The catalysts to be used according to this embodiment are oxidoreductases or other enzymes that are capable of oxidation in the presence of a suitable redox system. Oxido-reductases, i.e. enzymes capable of oxidation without the presence of further redox systems, to be used include peroxidases and oxidases, in particular polyphenol oxidases and laccase. Certain hydrolases, such as phytase, can be used when a further redox system is present such as a metal complex, e.g. vanadate. Metal complexes as such, without an enzyme protein, can also be used; examples include copper and iron complexes with porphyrins, phenanthrolins, polyamines such as EDTA, EGTA and the like. The metal-assisted enzymes and metal complexes require hydrogen peroxide, alkyl and ar(alk)yl hydroperoxides (such as tert-butyl hydroperoxide) or chlorite as an ultimate electron acceptor. Peroxidases (EC 1.11.1.1 - 1.11.1.11) that can be used according to the invention include the peroxidases which are cofactor-independent, in particular the classical peroxidases (EC 1.11.1.7). Peroxidases can be derived from any source, including plants, bacteria, filamentous and other fungi and yeasts. Examples are horseradish peroxidase, soy-hull peroxidase, myelo peroxidase, lactoperoxidase, Arthromyces and Coprinus peroxidases. Several peroxidases are commercially available. The peroxidases require hydrogen peroxide as an electron acceptor. Polyphenol oxidases (EC 1.10.3.1.) include tyrosinases and catechol oxidases such as lignine peroxidase. Suitable polyphenol oxidases may be obtained from fungi, plants or animals. The polyphenol oxidases require oxygen as an electron acceptor. Laccases (EC 1.10.3.2) are sometimes grouped under the polyphenol oxidases, but they can also be classified as a distinct group, sometimes referred to as p-diphenol oxidases. The laccases can be derived from plant sources or from microbial, especially fungal, sources. The laccases also require oxygen as an electron acceptor. The process for producing the aldehydes according to this embodiment can be performed under relatively mild conditions, e.g. at a pH between 2 and 10, and at a temperature between 15 and 60°C (both depending on the particular enzyme or metal complex). The reaction medium can be an aqueous medium, or a homogeneous mixed medium, e.g. a mixture of a water and a water-immiscible organic solvent such as a hydrophobic ether, a hydrocarbon or a halogenated hydrocarbon. In the latter case, the enzyme and/or the nitroxyl and the oxidising agent may be present in the aqueous phase and the hydroxy compound substrate and the aldehyde or ketone product may be present in the organic phase. If necessary, a phase transfer catalyst may be used.
The reaction medium can also be solid/liquid mixture, in particular when the enzyme of the nitroxyl are immobilised on solid carrier. A heterogeneous reaction medium may be advantageous when the substrate or the product is relatively sensitive or when separation of the product from the other reagents may present difficulties.

According to the present invention it is preferred to conduct step (i), i.e. the oxidation of a hydroxy compound, with cellulosic pulp as substrate in the presence of the above enzyme as primary oxidant and catalytic amounts of a nitroxy mediator, preferably those of formula (V), such as TEMPO or 4-acetamido TEMPO.

Further in particular amido-substituted nitroxy compounds (e.g. 4-acetamido TEMPO) of the aforementioned formula V are suitable for stoichiometric oxidation at a pH between 1 and 7, preferably 2 to 6, more preferably 2 to 3. In this reaction, halogen-free acids such as sulfuric acid or toluene sulfonic acid are particularly suitable for adjusting the pH.
Under these conditions, the nitroxy compound present as a radical disproportionates into an oxammonium ion that acts as an oxidizing species and a hydroxylamine. After the reaction, the consumed (reduced) form of the nitroxy compound can be regenerated with ozone or another oxidizing agent, preferably in a separate process step. An important advantage of the oxidation version discussed here is the ability to use the choice of a suitable pulp (TCF, see following sections) to conduct the entire pulp or paper production method without any halogen-containing chemicals.

Regarding suitable reaction conditions for conducting the nitroxy-mediated oxidation of a hydroxy compound, reference can also be made to WO 95/07303, WO 99/57158, WO 99/58574, WO 00/26257, WO 00/50621, WO 00/50388, WO 00/50463, WO 01/00681, WO 00/50462, WO 01/34656, WO 01/34903, WO 01/34657, WO 01/83887, EP 1,149,846, EP 1,215,217 A and EP 1,251,140 A in the name of TNO and SCA Hygiene Products GmbH (AB, Zeist B.V).

The oxidation reaction of step (i) is conducted in an aqueous reaction medium or a mixture of water and a water-miscible organic solvent as previously described (preferred volume ratio water/other solvent of more than 50%, in particular at least 80% water). Suitable organic solvents are for instance selected from non-alcoholic, water-miscible solvents such as ketones (e.g. acetone), ethers (e.g. THF (tetrahydrofuran) or dioxane), or nitriles (e.g. CH₃CN).

If water is used as reaction medium, the organic nitroxy compound will always show a sufficient affinity to the hydrophobic resin in the following separation step due to strong interactions between the resin surface and hydrophobic molecule parts of the nitroxy compound. Regarding mixtures of water and a water-miscible organic solvent, the content of organic solvent should not exceed limits reducing or preventing the retention of the nitroxy compound on the hydrophobic resin in the subsequent step (ii). This depends primarily on the type of nitroxy compound, resin and organic solvent. In this respect, a skilled person is capable of selection suitable conditions including an appropriate ratio of water/organic solvent.

According to **step (ii)** of the claimed oxidation process, unreacted nitroxy compound remaining in the reaction mixture obtained in step (i) is separated from other ingredients (starting materials, products, primary oxidant, other catalyst, etc) by contacting the reaction mixture with a hydrophobic resin. This step can be conducted in line with the teaching of WO 02/059064.

Suitable resins can be selected from non-ionic, hydrophobic, preferably crosslinked resins. These resins preferably contain, as major monomeric unit in terms of molar amount, polymerizable aromatic monomeric units such as styrene or divinylbenzene or vinyl monomers such as (meth)acrylic acid. Crosslinking may take place during and/or after polymerisation. Suitable resins preferably display a macroreticular structure involving both a continuous polymer phase and a continuous pore phase. The harmonic mean size of the resin beads preferably ranges from 0,4 to 0,7 mm. Furthermore, it is preferred if the surface area is above 380 m²/g and/or the porosity above 0,5 ml/ml.

Such resins are available under the trade name XAD® from Sigma, USA; Supelco, Bellefonte, PA, USA or Rohm & Haas company, USA. Suitable types are for instance XAD-2, XAD-4, XAD-8, XAD-11, XAD-16, XAD-30, or XAD-1180.

According to step (ii), the reaction mixture obtained in the oxidation step (i) is brought into contact with the hydrophobic resin either batchwise or preferably by means of a chromatographic separation.

Generally, the organic nitroxy compound will show a stronger affinity to the hydrophobic resin if it is present in an aqueous solution or in a mixture of water and water-miscible organic solvent having a relatively high water content (e.g. at least 80, preferably at least 90 Vol.%). This contacting step is preferably conducted at lower temperatures ranging from 10°C to 50 °C, for instance 10 to 30°C.

**Step (iii)** then requires removing the nitroxy compound from the hydrophobic resin by elution with a preferably water-miscible organic solvent or a suitable mixture of water and a water-miscible organic solvent. The water-miscible organic solvent is preferably selected among the already described ones. At any rate, it is necessary that the eluent is less polar (more hydrophobic) than the reaction mixture from which the nitroxy compound was adsorbed. Thus, organic solvents such as ethanol, 1- or 2-propanol, t- butanol, acetone, THF dioxane, CH₃CN, or their mixtures with water, are capable of eluting nitroxy compounds that were retained from an aqueous reaction mixture. If the eluting organic solvent is an alcohol, its oxidation by the organic nitroxy compound is of course to be prevented, for instance by lowering the pH value and thereby reducing the reaction rate.

Similarly, a mixture of water and water-miscible organic solvent having a higher content of organic solvent (per volume ratio, e.g. at least 5% or 10% more) is basically suitable to elute organic reaction nitroxy compounds that were retained from a mixture of water and organic solvent having a lower content of organic solvent. A suitable difference in terms of volume ratio can be easily determined by a skilled person.

The elution step (iii) involves washing the hydrophobic resin, on which the organic nitroxy compound is absorbed, with a suitable solvent (mixture) in the case of batchwise operation. If step (iii), on the other hand, is to be conducted as chromatographic separation, the organic nitroxy compound is eluted from the column by passing the organic solvent over the same.

Similar to step (ii), step (iii) is conducted preferably at relatively low temperatures ranging from 10 to 30°C.

In line with **step (iv)**, the nitroxy compound is then recovered from this organic solution containing the pure nitroxy compound in a manner claimed and described for the recovery process of the present invention.

**Step (v)** of the oxidation process finally involves recycling the organic nitroxy compound obtained thereby into step (i). This last step provides an economically advantageous closed cycle oxidation process. This process minimizes catalyst losses since the solvent removal steps are carried out in the presence of ionic, non-volatile compounds, i.e. nitrosonium ions and protonated hydroxylamine.

According to a preferred embodiment of the claimed oxidation process, a hydroxy compound, preferably an oligo- or polysaccharide, such as starch or cellulose is oxidized in the presence of an organic nitroxy compound, in particular TEMPO and its derivatives, as catalyst and an oxidising enzyme as primary oxidant. It is most preferred, if this process is used for oxidising, at least in part, the hydroxy groups occurring in cellulosic pulp to aldehyde and/or carboxy groups.

The present invention is now further illustrated by the following examples:

### Examples

### Example 1:

2g TEMPO were dissolved in 50 ml acetone. To the resulting yellow-orange-colored solution, 5 ml 8M-hydrochloric acid was added. The solution turned dark brown. After standing for 15 minutes, the solvent was removed by vacuum distillation in a rotary evaporator. The resulting residue was analysed by UV-vis spectroscopy. The analysis showed that the resulting residue did no longer contain any nitroxy functionality λ max of TEMPO = 425 mm). TEMPO was rather completely converted into the protonated hydroxylamine and the nitrosonium ion (λ max at 460-480 nm).

The residue was then dissolved in water followed by raising the pH to 6. Again, a yellow-orange-colored solution formed and the UV-vis spectroscopic analysis confirmed that TEMPO (nitroxy compound) was again present as sole agent.

### Example 2:

An aqueous solution of TEMPO (5g/l) was passed over a column filled with 30 g XAD 1180 resin (available from Supelco, Belfonte, USA). After passing 120 ml of this solution over the column, the same was saturated (binding capacity of the resin is 2 wt% TEMPO). The column was subsequently eluted with acetone to remove TEMPO. The colored fractions were collected, acidified with hydrochloric acid (pH 0.2) to obtain a brownish solution. The solvent was removed by vacuum distillation using a rotary evaporator. As the distilled liquid was colorless, in contrast to non-acidified TEMPO-containing solutions, it can be concluded that there is no loss of TEMPO. The residue (yellow-brown solid) was dissolved in water and neutralized leading to the formation of TEMPO, as was confirmed by UV-Vis spectroscopy.

### Example 3:

An aqueous solution of TEMPO was prepared (C=2000 mg/l). The absorbance at λ =430 nm of two (blanc) solutions was measured. 250 ml of solution No. 1 was passed over a column containing 30g XAD 1180 resin. The TEMPO bound to the column was released with 50 ml acetone. The TEMPO-containing acetone solution was acidified with 20 ml diluted hydrochloric acid (1.5M) and was allowed to stand for 30 minutes. Afterwards excess of acetone was removed under reduced pressure. The residue was dissolved in water and neutralised with sodium hydroxide (1M) and diluted with water to 250ml (solution No. 3). Next the E₀ at 430 nm was measured with UV-Vis. This procedure was repeated with solution No. 2 leading to the recovered solution No. 4. The results of the respective measurements are given in Table 1.

**Table 1. Recovery of TEMPO from an XAD 1180 column.**

| Sample | E_{430 nm} | Recovery (%) |
|---|---|---|
| Solution No 1 (blanc) | 0.166 | |
| Solution No 2 (blanc) | 0.166 | |
| Solution No 3 (recovered) | 0.167 | 100.6 |
| Solution No 4 (recovered) | 0.165 | 99.4 |

Taking into account the analytical errors it thus appears that the recovery is quantitative (100.0 ± 0.6%).

## Claims

1. Process for the recovery of an organic nitroxy compound from a solution of this nitroxy compound in an organic solvent or a mixture of a water-miscible organic solvent and water, said process comprising the steps of:
(a) adjusting the pH of said solution to a value below 4 to generate a nitrosonium ion and the protonated hydroxylamine
(b) removing the organic solvent until a residue containing the nitrosonium ion and the protonated hydroxylamine has formed,
(c) if necessary, redissolving the residue obtained in water, and
(d) neutralizing the resulting aqueous solution to recover the nitroxy compound.

2. Process according to claim 1 wherein the nitroxy compound has the following formula (V) 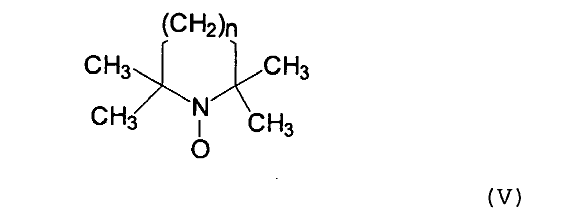 where n = 0 or 1 and where the methylene groups of the ring may carry one or more substituents selected from alkyl, alkoxy, aryl, aryloxy, amino, amido, oxo, cyano, hydroxy, carboxyl, phosphonooxy, maleimido, isothiocyanato, alkyloxy, fluorophosphinyloxy, substituted or unsubstituted benzoyloxy.

3. Process according to claim 1 wherein the organic solvent is selected from water-miscible alcohols, ethers, ketones and nitriles.

4. Process according to claim 1 wherein the acid is selected from non-oxidizing and non-reducing organic or inorganic acids.

5. Process according to claim 4 wherein the acid is toluenesulfonic acid, benzenesulfonic acid, hydrochloric acid or sulfuric acid.

6. Process according to claim 1, wherein the solution of the nitroxy compound is obtained by eluting the nitroxy compound from a chromatographic column filled with a hydrophobic resin.

7. Process for the oxidation of a hydroxy compound in the presence of a nitroxy compound comprising the steps of
(i) oxidizing a hydroxy compound in the presence of a nitroxy compound and optionally a primary oxidant in a water-containing reaction medium,
(ii) separating the nitroxy compound remaining in the reaction mixture obtained thereby by contacting the reaction mixture with a hydrophobic resin,
(iii)removing the nitroxy compound from the hydrophobic resin by eluting the same with an organic solvent or a mixture of water and a water-miscible organic solvent, said mixture having a lower polarity than the reaction medium,
(iv) recovering the nitroxy compound from the resulting solution by the process according to any of claims 1 to 6,
(v) recycling the nitroxy compound obtained thereby into step (i).

8. Process according to claim 7, wherein step (ii) is conducted by passing the reaction mixture over a chromatographic column filled with a hydrophobic resin.

9. Process according to claim 7 or 8, wherein the hydrophobic resin is selected from XAD resins.

10. Process according to any of claims 7 to 9 wherein in step (i) at least some primary hydroxy groups of cellulosic pulp are oxidized in the presence of an enzyme as primary oxidant to aldehyde and/or carboxy groups.

11. Process according to any of claim 7 to 9 wherein in step (i) at least some primary hydroxy groups of cellulosic pulp are oxidized in the absence of chlorine-containing oxidants.

## Patentansprüche

1. Verfahren zur Rückgewinnung einer organischen Nitroxyverbindung aus einer Lösung dieser Nitroxyverbindung in einem organischen Lösungsmittel oder einer Mischung eines wassermischbaren organischen Lösungsmittels und Wasser, wobei das Verfahren die Schritte umfaßt:
(a) Einstellen des pH-Werts der Lösung auf einen Wert unter 4, um ein Nitrosoniumion und das protonierte Hydroxylamin zu bilden,
(b) Entfernen des organischen Lösungsmittel, bis ein Rückstand gebildet worden ist, der das Nitrosoniumion und das protonierte Hydroxylamin enthält,
(c) falls notwendig, Wiederauflösen des erhaltenen Rückstands in Wasser und
(d) Neutralisieren der resultierenden wäßrigen Lösung, um die Nitroxyverbindung zurückzugewinnen.

2. Verfahren gemäß Anspruch 1, worin die Nitroxyverbindung die folgende Formel (V) aufweist: 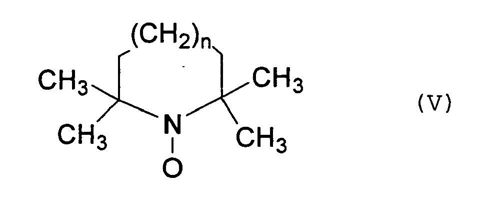 worin n = 0 oder 1, und worin die Methylengruppen des Rings einen oder mehrere Substituenten, ausgewählt aus Alkyl, Alkoxy, Aryl, Aryloxy, Amino, Amido, Oxo, Cyano, Hydroxy, Carboxyl, Phosphonooxy, Maleimido, Isothiocyanato, Alkyloxy, Fluorphosphinyloxy, substituiertem oder unsubstituiertem Benzoyloxy, aufweisen können.

3. Verfahren gemäß Anspruch 1, worin das organische Lösungsmittel aus wassermischbaren Alkoholen, Ethern, Ketonen und Nitrilen ausgewählt ist.

4. Verfahren gemäß Anspruch 1, worin die Säure aus nichtoxidierenden und nicht-reduzierenden organischen oder anorganischen Säuren ausgewählt ist.

5. Verfahren gemäß Anspruch 4, worin die Säure Toluolsulfonsäure, Benzolsulfonsäure, Salzsäure oder Schwefelsäure ist.

6. Verfahren gemäß Anspruch 1, worin die Lösung der Nitroxyverbindung durch Eluieren der Nitroxyverbindung von einer Chromatographiesäule, die mit einem hydrophoben Harz gefüllt ist, erhalten wird.

7. Verfahren zur Oxidation einer Hydroxyverbindung in der Gegenwart einer Nitroxyverbindung, umfassend die Schritte:
(i) Oxidieren einer Hydroxyverbindung in Gegenwart einer Nitroxyverbindung und gegebenenfalls eines primären Oxidationsmittels in einem wasserhaltigen Reaktionsmedium,
(ii) Abtrennen der Nitroxyverbindung, die in der hierdurch erhaltenen Reaktionsmischung verbleibt, indem die Reaktionsmischung mit einem hydrophoben Harz in Kontakt gebracht wird,
(iii) Entfernen der Nitroxyverbindung von dem hydrophoben Harz durch Eluieren desselben mit einem organischen Lösungsmittel oder einer Mischung aus Wasser und einem wassermischbaren organischen Lösungsmittel, wobei die Mischung eine geringere Polarität als das Reaktionsmedium besitzt,
(iv) Zurückgewinnen der Nitroxyverbindung aus der resultierenden Lösung durch das Verfahren gemäß irgendeinem der Ansprüche 1 bis 6,
(v) Zurückführen (Recyceln) der hierdurch erhaltenen Nitroxyverbindung in Schritt (i).

8. Verfahren gemäß Anspruch 7, worin Schritt (ii) durchgeführt wird, indem die Reaktionsmischung durch eine Chromatographiesäule geführt wird, die mit einem hydrophoben Harz gefüllt ist.

9. Verfahren gemäß Anspruch 7 oder 8, worin das hydrophobe Harz aus XAD-Harzen ausgewählt ist.

10. Verfahren gemäß irgendeinem der Ansprüche 7 bis 9, worin in Schritt (i) zumindest einige primäre Hydroxygruppen von zellulosem Zellstoff ("cellulosic pulp") in Gegenwart eines Enzyms als primärem Oxidationsmittel zu Aldehyd- und/oder Carboxygruppen oxidiert werden.

11. Verfahren gemäß irgendeinem der Ansprüche 7 bis 9, worin in Schritt (i) zumindest einige primäre Hydroxygruppen von zellulosem Zellstoff ("cellulosic pulp") in Abwesenheit von chlorhaltigen Oxidationsmitteln oxidiert werden.

## Revendications

1. Procédé permettant de récupérer un composé nitrosé organique à partir d'une solution de ce composé nitrosé dans un solvant organique ou un mélange d'eau et d'un solvant organique miscible à l'eau, lequel procédé comporte les étapes suivantes :
a) ajuster le pH de ladite solution à une valeur inférieure à 4, pour former un ion nitrosonium et une hydroxylamine protonée ;
b) chasser le solvant organique jusqu'à obtenir un résidu contenant l'ion nitrosonium et l'hydroxylamine protonée ;
c) si nécessaire, redissoudre dans de l'eau le résidu ainsi obtenu ;
d) et rendre neutre la solution aqueuse résultante, afin de récupérer le composé nitrosé.

2. Procédé conforme à la revendication 1, dans lequel le composé nitrosé présente la formule (V) suivante : 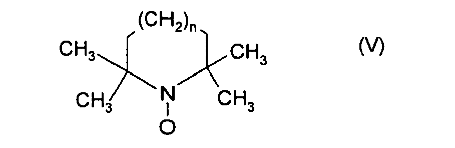 dans laquelle l'indice n vaut 0 ou 1, et les groupes méthylène du cycle peuvent porter un ou plusieurs substituant(s) choisi(s) parmi les substituants alkyle, alcoxy, aryle, aryloxy, amino, amido, oxo, cyano, hydroxy, carboxy, phosphonoxy, maléimido, isothiocyanato, alkyloxy, fluorophosphinyloxy, et benzoyloxy avec ou sans substituant(s).

3. Procédé conforme à la revendication 1, dans lequel le solvant organique est choisi parmi les alcools, éthers, cétones et nitriles miscibles à l'eau.

4. Procédé conforme à la revendication 1, dans lequel l'acide est choisi parmi les acides organiques ou inorganiques, non-oxydants et non-réducteurs.

5. Procédé conforme à la revendication 4, dans lequel l'acide est de l'acide toluènesulfonique, de l'acide benzènesulfonique, de l'acide chlorhydrique ou de l'acide sulfurique.

6. Procédé conforme à la revendication 1, dans lequel la solution de composé nitrosé est obtenue par élution du composé nitrosé dans une colonne de chromatographie garnie d'une résine hydrophobe.

7. Procédé d'oxydation d'un composé hydroxylé en présence d'un composé nitrosé, comportant les étapes suivantes :
i) oxyder un composé hydroxylé en présence d'un composé nitrosé et, en option, d'un oxydant primaire, dans un milieu réactionnel contenant de l'eau ;
ii) séparer le composé nitrosé qui reste dans le milieu réactionnel ainsi obtenu, en mettant ce mélange résctionnel en contact avec une résine hydrophobe ;
iii) séparer le composé nitrosé d'avec la résine hydrophobe, en l'éluant à l'aide d'un solvant organique ou d'un mélange d'eau et d'un solvant organique miscible à l'eau, ce mélange étant moins polaire que le milieu réactionnel ;
iv) récupérer le composé nitrosé à partir de la solution résultante, par un procédé conforme à l'une des revendications 1 à 6 ;
v) et recycler dans l'étape (i) le composé nitrosé ainsi obtenu.

8. Procédé conforme à la revendication 7, dans lequel on effectue l'étape (ii) en faisant passer le mélange réactionnel dans une colonne de chromatographie garnie d'une résine hydrophobe.

9. Procédé conforme à la revendication 7 ou 8, dans lequel la résine hydrophobe est choisie parmi les résines de type XAD.

10. Procédé conforme à l'une des revendications 7 à 9, dans lequel, dans l'étape (i), ce sont au moins certains des groupes hydroxyle primaires d'une pâte cellulosique que l'on oxyde, en présence d'une enzyme servant d'oxydant primaire, en groupes formyle et/ou carboxyle.

11. Procédé conforme à l'une des revendications 7 à 9, dans lequel, dans l'étape (i), ce sont au moins certains des groupes hydroxyle primaires d'une pâte cellulosique que l'on oxyde, en l'absence d'oxydants chlorés.
